# EUROPEAN PATENT APPLICATION

(11) **EP 3 734 276 A1**
(43) Date of publication of application: **04.11.2020**
(21) Application number: 17936951.7
(22) Date of filing: 28.12.2017
(51) Int. Cl.: G01N 33/53, G01N 33/48

(54) **BLOCKING REAGENT**

(71) Applicant: Nichirei Biosciences Inc., Chuo-ku Tokyo 104-8402 (JP)
(72) Inventor: KITANO Yuriko, Higashimurayama-shi, Tokyo 189-0003 (JP); FUJITA Kayoko, Tokyo 104-8402 (JP); IWAMOTO Yu, Higashimurayama-shi, Tokyo 189-0003 (JP)
(74) Representative: Plasseraud IP
(86) International application number: PCT/JP2017/047298
(87) International publication number: WO 2019/130560

(57) **Abstract**

[Problem]

To provide a blocking agent having an enhanced effect to prevent coloring of a slide glass in immunohistochemical staining.

[Solution]

A blocking reagent for immunohistochemical staining containing succinylated BSA as an active ingredient.

## Description

### BACKGROUND OF INVENTION

### Technical Field

The present invention relates to a blocking reagent used for immunohistochemical staining.

### Background Art

The immunohistochemical staining is a staining method to visualize a specific antigen using an antigen-antibody reaction on a tissue section and is commonly used as effective measures for the morphological study of biological tissue. The immunohistochemical staining generally adopts the following procedure: attaching a pathological tissue sample and an immunohistochemical specimen on a slide glass, performing various stainings, and observing with a microscope. The surface of the slide glass used here is coated with poly-L-lysine, 3-aminopropyltriethoxysilane (APS), a MAS coat (Matsunami glass Ind., Ltd.), and the like to prevent peel-off of cells and a tissue section from the slide glass. The preventing effect of the peel-off of the section is achieved by adhering a cell surface, which is basically negatively charged, to a glass surface that is positively charged by an amino group of the coating material.

The immunohistochemical staining method generally includes the following steps: deparaffinizing, reacting a first antibody, reacting a detection reagent and reacting a color reagent. Use of the color reagent may cause a phenomenon in which the slide glass is colored by a developing pigment in the reagent, which is frequently observed particularly when using an automatic immunostaining device. The coloring of the slide glass causes a problem in which a part of a specimen, particularly a surrounding part of an observation object is observed as colored, and thus a part that should not be colored under normal conditions is observed as colored.

To date, countermeasures are taken such as adding a blocking agent, represented by bovine serum albumin (BSA), to a first antibody and a detection reagent, and further an improved technique is devised (PTL 1 and 2), but coloring of the slide glass cannot be completely prevented, and presently further improvement is expected.

### PRIOR ART DOCUMENT

### PATENT DOCUMENT

[Patent Document 1] JP 2004-219111 A
[Patent Document 2] JP H7-218508 A

### SUMMARY OF INVENTION

The present inventors found that use of succinylated BSA as a blocking agent instead of conventionally-used bovine serum albumin (BSA) can reduce coloring of the slide glass in immunohistochemical staining. The present invention is based on this finding.

Therefore, an object of the present invention is to provide a blocking agent having an enhanced effect to prevent coloring of the slide glass in immunohistochemical staining.

The present invention includes the following inventions:
(1) A blocking reagent for immunohistochemical staining including succinylated BSA as an active ingredient.
(2) The blocking reagent for immunohistochemical staining according to above (1), wherein the active ingredient is dissolved in a buffer solution of pH 6.2 to 6.7.
(3) The blocking reagent for immunohistochemical staining according to above (1) or (2), wherein the active ingredient is dissolved in a MOPS buffer solution.
(4) A method for immunohistochemical staining, characterized by using the blocking reagent for immunohistochemical staining according to any one of above (1)-(3).

According to the present invention, coloring of a slide glass can be suppressed significantly by preventing a detection reagent from binding to the slide glass surface charged positively. In addition, the coloring of a slide glass can be caused by binding a first antibody to the slide glass surface as well as by binding a detection reagent directly to the slide glass surface, and, according to the present invention, an enhanced effect to separate a first antibody nonspecifically bound to a slide glass is confirmed. Thus, the present invention is advantageous in enabling to significantly inhibit coloring of a slide glass due to nonspecific binding of the first antibody.

### DETAILED DESCRIPTION OF THE INVENTION

The blocking reagent of the present invention includes succinylated BSA (succinylated bovine serum albumin) as an active ingredient.

The active ingredient used in the present invention is obtained by succinylating BSA and thus has a carboxyl group (-COOH) on the amino group, and specifically -NH₂ (amino group) is converted to a structure of -NH-CO-CH₂-CH₂-COOH. Although not intended to be limited by specific theory, since the carboxyl group is dissociated and negatively charged in a buffer solution, the above-mentioned active ingredient, when used with a detection reagent, exhibits higher affinity to a slide glass surface charged positively than the detection reagent, and, as a result, it is considered to prevent binding of the detection reagent to the slide glass surface. The active ingredient is considered also to enable separation of a first antibody, once bound to the slide glass, by means of the high affinity of the ingredient to the slide glass surface.

The succinylation of BSA can be carried out by a method well-known to those skilled in the art. For example, BSA can be succinylated by adding succinic anhydride to an aqueous BSA solution and stirring the solution under alkaline condition (e.g., pH 8.0) at room temperature.

The succinylation of BSA can be confirmed by assaying the residual amino groups. The assay can be carried out by using a method well-known as the assay of amino groups in a protein to those skilled in the art. For example, since 2,4,6-trinitrobenzenesulfonate sodium dihydrate (TNBS) specifically reacts with an amino group and produce a trinitrophenyl (TNP) derivative having an absorption maximum at 340-350 nm, amino groups can be assayed by measuring absorbance at 340-350 nm after the above-mentioned specific reaction. In this case, amino groups can be assayed precisely by using an amino acid having one amino group such as glycine as a reference material, making a standard curve using samples having different concentrations of the reference material, and comparing a measured value with this standard curve. According to a preferable embodiment of the present invention, the active ingredient used in the present invention was analyzed by the above-mentioned method using TNBS to obtain the concentration of amino acid, which is converted to about 0 µg/ml, and therefore the number of amino groups in the active ingredient used in the present invention is to be less than 1, by using the above-mentioned method.

The active ingredient in the present invention can be brought into contact with a tissue and a slide glass in the form as dissolved in a liquid. Here, the concentration of the active ingredient in the liquid may be similar to the concentration of conventionally-used BSA, and not limited in particular. For example, the active ingredient in the present invention can exert adequate blocking effect when dissolved in the liquid for applying to the tissue and slide glass at the concentration of 0.1% w/v or more, preferably 0.15% w/v or more, more preferably 0.35% w/v or more, and further preferably 0.5% w/v or more. In the active ingredient in the present invention, the more the amount is used, the better the blocking effect is achieved, and thus the use amount (concentration), though not particularly limited for the upper limit, appropriately is set at about 3.0% w/v in view of the amount of BSA used for normal immunohistochemical staining. According to a preferable embodiment of the present invention, the blocking reagent of the present invention is to include succinylated BSA as an active ingredient at the above-mentioned concentration.

The active ingredient in the present invention can be the ingredient dissolved in a buffer solution. The buffer solution may be any buffer solution, as long as it enables the carboxyl group in the active ingredient to be negatively charged, and not limited in particular. It can be, for example, a buffer solution of pH 6.2-6.7. In addition, type of the buffer solution is not limited in particular either, may be any type of buffer solution as long as it can be used for immunohistochemical staining, and is to be preferably a MOPS buffer solution. According to a preferable embodiment of the present invention, the active ingredient in the present invention is to be as dissolved in the MOPS buffer solution of pH 6.2-6.7.

According to a particularly preferable embodiment of the present invention, the active ingredient in the present invention is to be as dissolved in StabilZyme (registered trademark) HRP Conjugate Stabilizer (manufactured by SurModics Inc.). StabilZyme (registered trademark) HRP Conjugate Stabilizer contains a MOPS buffer solution of pH 6.2-6.7, in which BSA is dissolved.

The blocking agent of the present invention can be used suitably in immunohistochemical staining. The immunohistochemical staining to which the blocking agent of the present invention can be applied is not limited in particular, and may be any embodiment of immunohistochemical staining. Particularly, the blocking agent of the present invention can be suitably used in the immunohistochemical staining which uses a carrier (slide glass, and the like) which surface is positively charged.

The color development system used in a method of immunohistochemical staining is not limited in particular either, and any color development system can be used. The blocking agent of the present invention is suitably used, for example, for a commonly-used combination in immunohistochemical staining of peroxidase (horseradish peroxidase, and the like) and its chromogenic substrate.

The tissue section for immunohistochemical staining may be any of paraffin section or frozen section, and any tissue can be used. The blocking reagent of the present invention is effectively used particularly for blocking an organ/tissue composed of a complicated component in which nonspecific reactions are hardly inhibited.

### EXAMPLES

The present invention is explained specifically based on the following examples, but the present invention is not limited to these examples.

In the examples, the following protocols were used.

### 1. Succinylation

(1) 30 g of BSA (Sigma-Aldrich Inc.) is dissolved in 300 ml of distilled water.
(2) A pH electrode is set in a BSA solution of (1).
(3) 7.5 g of succinic anhydride (powder) is added by a small amount to the solution while the pH of the reaction solution is kept at 8.0 by adding a sodium hydroxide solution.
(4) After completion of the addition, the solution is stirred at room temperature for 30 minutes.
(5) A succinylated BSA solution of (4) is poured by half at a time into a dialysis membrane (Thermo Fisher Scientific Inc., Dialysis Membrane), and is dialyzed using distilled water overnight.
(6) The distilled water is replaced with new distilled water, and the solution is dialyzed overnight.
(7) The dialyzed succinylated BSA solution is filtered through 5.0 µm filter, and then through 0.45 µm filter.
(8) The resulting residue is freeze-dried for 5 days.
(9) The residual amino group is assayed.

### 2. Measurement of the residual amino group

(1) Standard (STD) and sample solutions are prepared.
   STD: Glycine 30, 20, 10, 5, 0 µg/ml
   Sample: Succinylated BSA 20, 10 mg/ml
   Buffer: 4% sodium hydrogen carbonate buffer (pH 8.5)
(2) To 1 ml each of STD and sample solutions, added is a 1 ml of 0.1% TNBS solution, and the solution is subjected to a reaction at 40°C for 2 hours.
(3) To the resulting solution, added are 1 ml of a 10% SDS solution and 0.5 ml of 1 N HCl, and the mixture is measured for its absorbance at 335 nm.
(4) A standard curve is made using a STD solution with 0 µg/ml of glycine as a blank. The number of amino groups of the succinylated BSA is determined (the number of amino groups in glycine is set to 1).

The measured value of succinylated BSA is converted by using the standard curve to obtain a concentration of amino acid of about 0 µg/ml, then the number of amino groups after the succinylation is confirmed to be less than 1, and therefore it is confirmed that the amino groups are completely substituted with carboxyl group.

### 3. Deparaffinization processing and antigen retrieval processing (normal stomach tissue)

(1) To a slide glass (manufactured by Matsunami glass Ind., Ltd., MAS coat slide glass), attached was a paraffin-embedded section (section thickness 3 µm) of a normal tissue of stomach, the slide glass was left standstill in a xylene layer for 3 minutes × 3 times, and thus the section was deparaffinized.
(2) Then, the slide glass was left standstill in an ethanol layer for 3 minutes × 4 times to be hydrophilized.
(3) After the last standstill in the ethanol layer, the slide glass was washed for 3 minutes × 3 times using a phosphate buffer solution (pH 7.6).
(4) In a plastic Coplin jar containing an EDTA-containing Tris-HCI buffer solution (pH 9.0), set was the slide glass already subjected to deparaffiniztion processing, and the jar was placed in a desk autoclave (manufactured by ALP Co., Ltd.), and subjected to antigen retrieval processing at 121°C for 20 minutes.
(5) After the antigen retrieval processing in the autoclave, the pressure in the machine was confirmed to be lowered, then the plastic Coplin jar was taken out, and left standstill at room temperature for 20 minutes.
(6) After the lapse of 20 minutes, the slide glass was placed in a phosphate buffer solution (pH 7.6) and washed at a standstill for 5 minutes × 3 times.

### 4. Deparaffinization processing and antigen retrieval processing (breast cancer tissue)

Deparaffinization processing and antigen retrieval processing were performed in the same method as the normal stomach tissue.

### 5. Immunohistochemical staining using an automatic immunostaining device

(1) After the heat treatment for antigen retrieval, the slide glass washed in the phosphate buffer solution (pH 7.6) was mounted in the slide rack of an automatic immunostaining device (HISTOSTAINER 36A, NICHIREI BIOSCIENCES INC.), and a phosphate buffer solution (pH 7.6) and the like was used to cover the tissue to prevent the tissue from drying.
(2) The slide glass mounted was washed using PBS (for HISTOSTAINER) (NICHIREI BIOSCIENCES INC.).
(3) After air drying, an aqueous 3% hydrogen peroxide solution (NICHIREI BIOSCIENCES INC.) was added dropwise to the slide glass mounted, which was subjected to a reaction for 5 minutes.
(4) After completion of the 5-minute reaction, the slide glass mounted was washed using PBS (for HISTOSTAINER) (NICHIREI BIOSCIENCES INC.).
(5) After air drying, in the case of breast cancer tissue, ER (1D5) mouse monoclonal diluted antibody (for HISTOSTAINER) (NICHIREI BIOSCIENCES INC.) was added dropwise to the slide glass, and in the case of normal stomach tissue, a phosphate buffer solution (pH 7.6) was added dropwise to the slide glass, which were subjected to a reaction at room temperature for 30 minutes.
(6) After air drying, to the slide glass, dropwise added was an HRP labeled goat anti-mouse polymer reagent (NICHIREI BIOSCIENCES INC.) prepared using each of dilute solutions according to the following examples, which was subjected to a reaction at room temperature for 30 minutes.
(7) After completion of the 30-minute reaction, the slide glass was washed twice using PBS (for HISTOSTAINER) (NICHIREI BIOSCIENCES INC.).
(8) To the slide glass, dropwise added was a DAB solution prepared using a DAB (a substrate of HRP for staining) substrate kit (for HISTOSTAINER) (NICHIREI BIOSCIENCES INC.), which was subjected to a reaction at room temperature for 10 minutes.
(9) After the 10-minute reaction, the slide glass was washed once using PBS (for HISTOSTAINER) (NICHIREI BIOSCIENCES INC.), and then once with water.
(10) The stained slide glass was taken out of the automatic immunostaining device, and washed under running water for 5 minutes.
(11) After the washing under running water, the slide glass (with tissue section) was drained, and subjected to a reaction with Mayer's hematoxylin for 30 seconds.
(12) After the 30-second reaction, the slide glass (with tissue section) was washed under running water for 5 minutes.
(13) After the washing under running water, the slide glass (with tissue section) was drained, and subjected to passage through an ethanol layer (3 times), leaving standstill in an ethanol layer (3 minutes, once), passage through a xylene layer (once), leaving standstill in a xylene layer (5 minutes, twice), and dehydration/clearing.
(14) The tissue section was encapsulated using a water-insoluble encapsulant (NICHIREI BIOSCIENCES INC.).

### [Example 1]

### Production of blocking agent

In accordance with a procedure of protocol 1, succinylated BSA was prepared. The prepared succinylated BSA was measured for the number of residual amino group in accordance with protocol 2 to obtain the number of amino groups of less than 1, and thus all amino groups were confirmed to be completely substituted with carboxyl group.

In addition, the prepared succinylated BSA was measured for its isoelectric point and the following results were obtained.
BSA: about 4.8
Succinylated BSA: about 4.7

BSA was succinylated and its isoelectric point shifted slightly to a lower pH. Therefore, it was inferred that, when dissolved in StabilZyme (registered trademark)-HRP (pH 6.2-6.7) used in the following examples, the succinylated BSA was negatively charged.

### [Example 2]

### Blocking effect against the non-specific staining to a tissue and slide glass

Effect for preventing non-specific staining to a tissue and slide glass in the automatic immunostaining device was compared between BSA, a conventional product, and the succinylated BSA which is functional group-modified BSA. Here, a first antibody was not used, and the blocking effect was confirmed against coloring due to binding of a polymer reagent directly to a tissue and slide glass.

This experiment was performed in accordance with protocol 3 and protocol 5. The main conditions of the immunohistochemical staining are as follows.
Automatic immunostaining device: HISTOSTAINER (NICHIREI BIOSCIENCES INC.)
First antibody: A first antibody was not used. Instead, a phosphate buffer solution (pH 7.6) was added dropwise.
Polymer reagent: An HRP labeled goat anti-mouse polymer reagent (NICHIREI BIOSCIENCES INC.) was diluted to 30 µg/mL with a solution prepared by dissolving BSA or succinylated BSA in StabilZyme (registered trademark) HRP (manufactured by SurModics Inc.) at the concentration shown in the following table, and each of the resulting solutions was subjected to a reaction at room temperature for 30 minutes. In order to reproduce a situation in which non-specific staining occurs easily, the concentration of anti-mouse-HRP polymer was set higher than under a normal staining condition.
Color development: DAB, 5 minutes
Coloring observation method: The coloring on a slide glass was observed visually. The non-specific staining to a tissue was observed at a magnification of 100-400 using an optical microscope BX53 manufactured by Olympus Corporation.

Results are shown in the following table 1.

**Table 1: Blocking effect of succinylated BSA against non-specific staining to a tissue/slide glass**

| Blocking agent | Concentration of blocking agent (% w/v) | Non-specific staining on tissue | Coloring on slide glass |
|---|---|---|---|
| None | 0 | ++ | ++' |
| BSA | 3.0 | + | ±' |
| | 1.5 | + | +' |
| | 0.75 | + | ++ |
| | 0.50 | + | ++ |
| | 0.375 | + | ++ |
| | 0.1875 | + | ++ |
| Succinylated BSA | 3.0 | ± | - |
| | 1.5 | ±' | - |
| | 0.75 | ±' | - |
| | 0.50 | ±' | ± |
| | 0.375 | ±' | ± |
| | 0.1875 | + | ± |

| | | | |
|---|---|---|---|
| Intensity of the non-specific staining/coloring: ++'> ++ > +' > + > ±' > ± > - As the number of "+" increases, coloring of slide glass becomes intense. "-" indicates that coloring of slide glass was not recognized at all. | | | |

As shown in table 1, when the polymer reagent contains only StabilZyme (registered trademark), intense non-specific staining was observed on the tissue. However, when blocking agents were added in the polymer agent, non-specific staining was inhibited. In addition, even when BSA was added as a blocking agent, non-specific staining was still confirmed, but when succinylated BSA was added as a blocking agent, non-specific staining was almost unconfirmable.

In addition, as for coloring on the slide glass, the sample using succinylated BSA as a blocking agent exerted higher effect on preventing coloring on the slide glass than the sample using BSA.

From the above results, succinylated BSA was confirmed to prevent the detection reagent from binding directly on the tissue and to exert superior effect on preventing detection of non-specific staining as compared with unmodified BSA. In addition, succinylated BSA was confirmed to prevent the detection reagent from binding directly on the slide glass and to exert elevated effect on preventing coloring as compared with unmodified BSA.

### [Example 3]

### Blocking effect against non-specific staining on a slide glass

Effect on preventing non-specific staining on a slide glass in the automatic immunostaining device was compared between BSA, a conventional product, and succinylated BSA which is functional group-modified BSA. Here, a first antibody was used and bound to a slide glass, and the blocking effect was confirmed against coloring of the slide glass caused by detection of the first antibody.

This experiment was performed in accordance with protocol 4 and protocol 5. The main conditions of the immunohistochemical staining are as follows.
Automatic immunostaining device: HISTOSTAINER (NICHIREI BIOSCIENCES INC.)
First antibody: Estrogen receptor (1D5) mouse monoclonal antibody (NICHIREI BIOSCIENCES INC.)
Polymer reagent: An HRP labeled goat anti-mouse polymer reagent (NICHIREI BIOSCIENCES INC.) was diluted to 10 µg/mL with a solution prepared by dissolving BSA or succinylated BSA in StabilZyme (registered trademark) HRP (manufactured by SurModics Inc.) at the concentrations shown in the following table, and each of the resulting solutions was subjected to a reaction at room temperature for 30 minutes.
Color development: DAB, 5 minutes
Coloring observation method: The coloring on a slide glass was observed visually.

Results are shown in the following table 2.

**Table 2: Blocking effect of succinylated BSA against non-specific staining of a slide glass via first antibody**

| Blocking agent | Concentration of blocking aqent (% w/v) | Coloring on slide glass |
|---|---|---|
| None | 0 | +++' |
| BSA | 3.0 | + |
| | 1.5 | + |
| | 0.75 | +' |
| | 0.375 | +++ |
| | 0.1875 | +++ |
| Succinylated BSA | 3.0 | - |
| | 1.5 | ± |
| | 0.75 | ± |
| | 0.375 | + |
| | 0.1875 | ++' |

| | | |
|---|---|---|
| Intensity of the non-specific staining/coloring: +++' > +++ > ++' > ++ > +' > + > ±' > ± > - As the number of "+" increases, coloring of slide glass becomes strong. "-" indicates that coloring of slide glass was not recognized at all. | | |

As shown in table 2, succinylated BSA was confirmed to exert elevated effect on preventing non-specific staining of a slide glass via a first antibody as compared with unmodified BSA.

## Claims

1. A blocking reagent for immunohistochemical staining comprising succinylated BSA as an active ingredient.

2. The blocking reagent for immunohistochemical staining according to claim 1, wherein the active ingredient is dissolved in a buffer solution of pH 6.2 to 6.7.

3. The blocking reagent for immunohistochemical staining according to claim 1 or 2, wherein the active ingredient is dissolved in a MOPS buffer solution.

4. A method for immunohistochemical staining, **characterized by** using the blocking reagent for immunohistochemical staining according to any one of claims 1 to 3.
